(19) Europäisches Patentamt
European Patent Office
Office européen des brevets

(11) Publication number: **0 053 038 A2**

# (12) EUROPEAN PATENT APPLICATION

(21) Application number: 81305548.0

(22) Date of filing: 24.11.81

(51) Int. Cl.³: C 07 C 119/18
C 07 C 125/08, C 07 C 161/00
C 07 D 239/47
//A01N47/36

(30) Priority: 25.11.80 US 210234

(43) Date of publication of application: 02.06.82 Bulletin 82/22

(84) Designated Contracting States: BE DE FR GB IT LU NL

(71) Applicant: E.I. DU PONT DE NEMOURS AND COMPANY
Legal Department 1007 Market Street
Wilmington Delaware 19898(US)

(72) Inventor: Rorer, Morris Padgett
64 Lower Valley Lane
Newark Delaware 19711(US)

(74) Representative: Hildyard, Edward Martin et al,
Frank B. Dehn & Co. European Patent Attorneys Imperial
House 15-19 Kingsway
London WC2B 6UZ(GB)

## (54) 3-Amino-2-alkenylimidates, their preparation and conversion to herbicide intermediates.

(57) Novel 3-amino-2-alkenylimidates of the formula

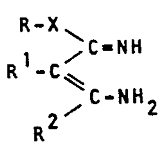

and the monohydrochloride and dihydrochloride thereof,
wherein

X is O or S,
R is alkyl, alkoxyalkyl or haloalkyl,
$R^1$ is H or alkyl,
$R^2$ is H or alkyl,

may be reacted with cyanamide or cyanogen chloride to yield novel cyanoimidates of the formula

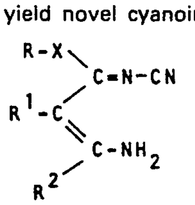

or

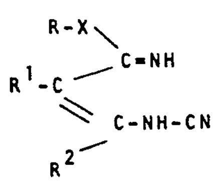

which in turn readily cyclize to pyrimidines of formula

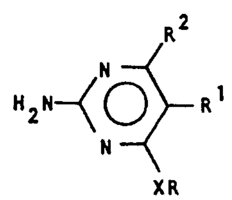

Both the pyrmidines and the cyanoimidates react with arylsulfonyl isocyanates to yield sulfonylurea herbicides.

EP 0 053 038 A2

Croydon Printing Company Ltd.

Title

3-Amino-2-alkenylimidates, their preparation and conversion to herbicide intermediates

Background of the Invention

This invention relates to novel 3-amino-2-alkenylimidates and to an improved route for preparing such 3-amino-2-alkenylimidates which, in turn, are important in preparing herbicide intermediates.

In our EP-A-24200 we have described processes involving bis-imidate intermediates, which are useful in preparing 2-amino-4,6-dialkoxypyrimidines. The dialkoxypyrimidines, in turn, are useful in preparing herbicidal sulfonamides.

The present invention relates to a process for preparing 2-amino-4-alkoxy-6-alkylpyrimidines which involves mono-imidate intermediates.

## Summary of the Invention

The present invention relates to the discovery of compounds of Formula I and II, to processes for their preparation and to their subsequent conversion to pyrimidines which are useful as herbicide intermediates.

### Compounds

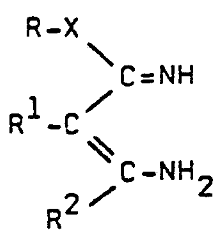

(I)

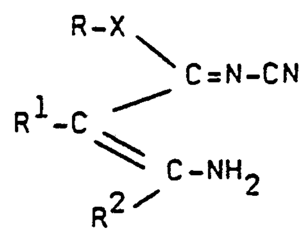

(II)

and the monohydrochloride and dihydrochloride thereof, wherein

X is O or S;
R is $C_1$-$C_4$ alkyl, $(CH_2)_n$-$OR^3$, $CH_2CH_2Cl$ or $CH_2CF_3$;
$R^1$ is H or $C_1$-$C_3$ alkyl;
$R^2$ is H or $C_1$-$C_3$ alkyl;
$R^3$ is $C_1$-$C_4$ alkyl; and
n is 1 or 2
provided that when R is $CH_2CH_2Cl$ or $CH_2CF_3$, then X is O.

Preferred for reasons of ease of synthesis or for the favorable utility of their resultant pyrimidine cyclization products as herbicide intermediates, or both, are those compounds of Formula I and II where

X is O;
$R^1$ is H; and
$R^2$ is $CH_3$.

<u>More preferred</u> for their more favorable ease of synthesis and for the highly favorable utility of their resultant pyrimidine cyclization products for preparing herbicides are those preferred compounds of Formula I and II in which

R is $C_1$-$C_4$ alkyl.

<u>Specifically preferred</u> for their most favorable ease of synthesis or for the greatest utility of the resultant pyrimidine cyclization product for preparing herbicides, namely, 2-amino-4-methoxy-6-methylpyrimidine, as a herbicide intermediate, are:

Methyl 3-amino-2-butenimidate dihydrochloride; and

Methyl 3-amino-N-cyano-2-butenimidate.

It will be understood that structures I and II can exist in the following tautomeric forms:

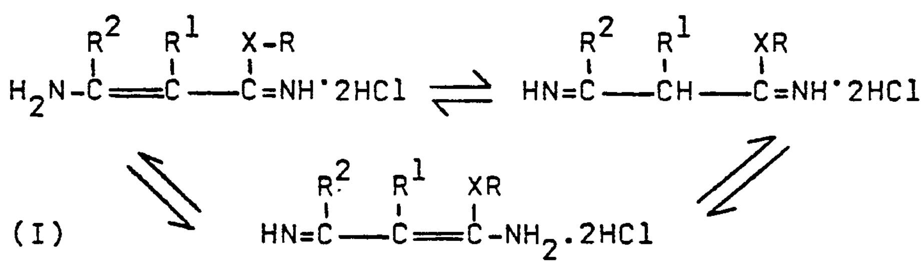

(I)

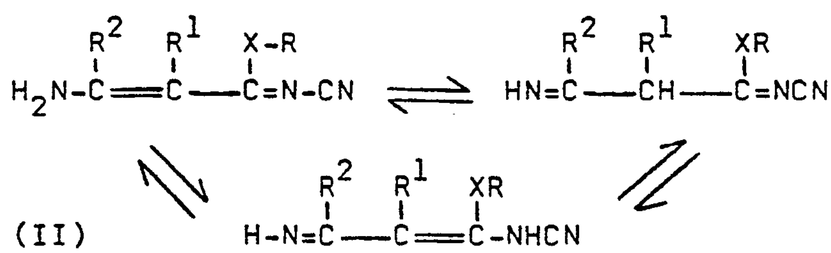

(II)

It will also be understood that some or all of intermediate II may exist as the structural isomer IIa

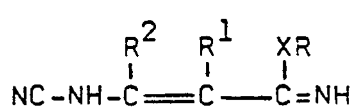

(IIa)

The ratio of IIa to II is not significant because both cyclize to the same pyrimidine intermediate.

Processes for preparing intermediates of Formula I, their subsequent conversion to intermediates of Formula II and their ultimate conversion to pyrimidines III which are useful as herbicide intermediates are shown below.

Scheme 1

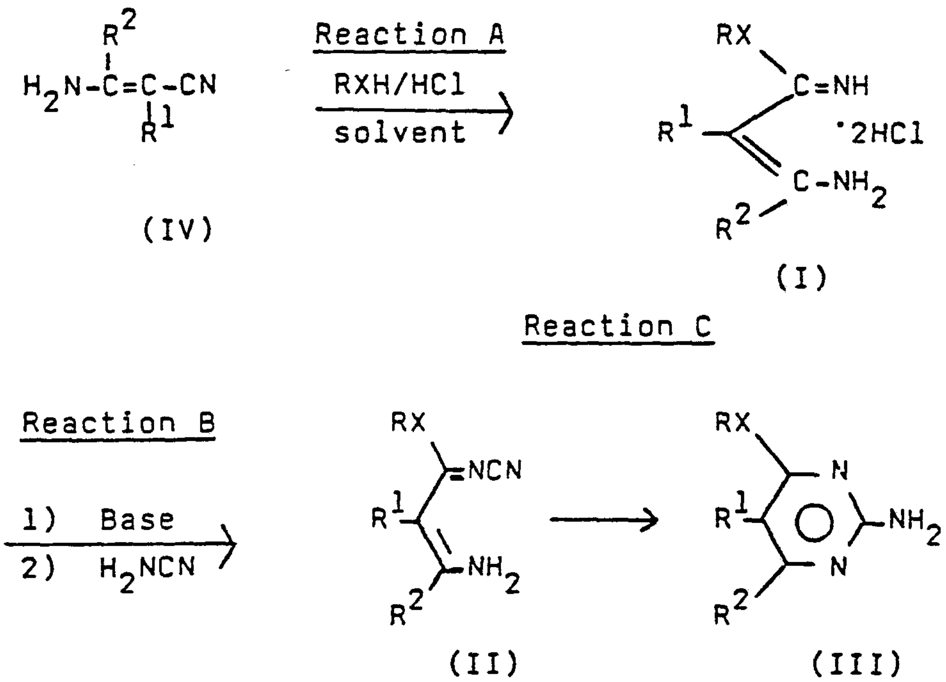


(IV) (I) (II) (III)

In Reaction A a substituted 3-aminocrotononitrile is contacted with an inert liquid medium saturated with hydrogen chloride gas and containing an excess, up to four moles, of alcohol or mercaptan. In Reaction B, the dihydrochloride salt of the 3-amino-2-alkenylimidate (I) is contacted with one equivalent of base. A 0-80% molar excess of cyanamide is then added, and the reaction is warmed and then cooled to produce (II). In Reaction C, (II) is converted to the pyrimidine (III) by heating.

Scheme 2

Reaction D

(I)

1) Base

2) $H_2NCN$

(III)

Alternatively, dihydrochloride salt (I) may be converted directly to the pyrimidine (III), as shown in Reaction D. Dihydrochloride salt (I) is contacted with one equivalent of a base followed by cyanamide and then heated to produce (III).

Scheme 3

Reaction E

(I)

1) $Base/H_2O/CH_2Cl_2$

2) ClCN

3) $\Delta$ T

(III)

As shown in Reaction E, an alternate process for preparing pyrimidine (III) consists of (a) contacting dihydrochloride salt (I) with two equivalents of a base to produce a free imidate, (b) contacting the imidate with cyanogen chloride to produce a cyanoimidate, and (c) isolating and heating the cyanoimidate to cause ring closure to (III).

Scheme 4

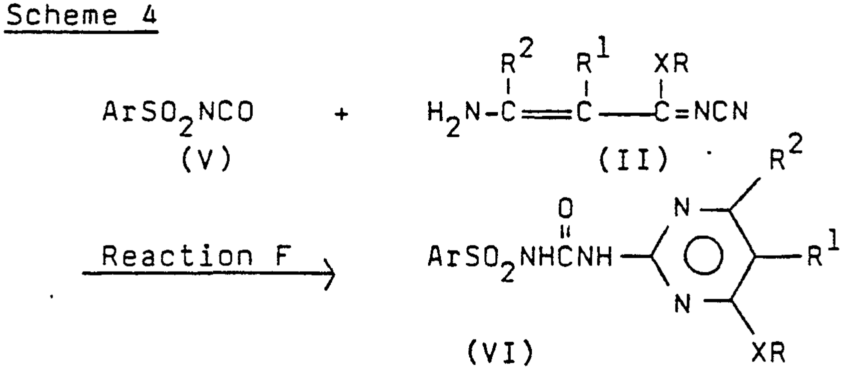


(V) (II) (VI)

The cyanoimidate (II) can be treated with one equivalent of an arylsulfonyl isocyanate (V) in an inert solvent to yield the corresponding sulfonylurea (VI), which is a selective herbicide.

Scheme 5

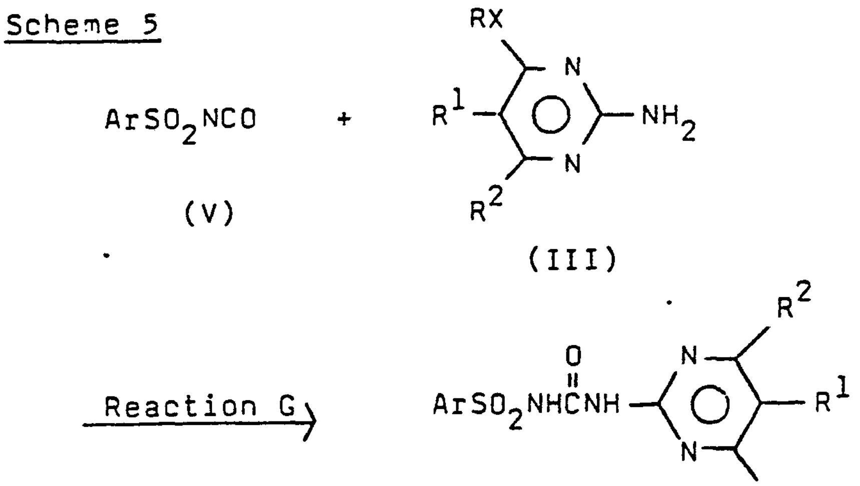


(V) (III) (VI)

According to Scheme 5, any pyrimidine (III), preformed or formed under the reaction conditions of Scheme 4, can also react with the arylsulfonylisocyanate (V) to form the corresponding sulfonylurea (VI).

Detailed Description of the Invention

The preferred processes used in preparing compounds of the invention (I) and (II), pyrimidines of Formula (III) and sulfonylureas of Formula (VI), illustrated heretofore in Reactions A through F, are more fully described as follows:

Reaction A illustrates the process for preparing compounds of the invention (I). According to this process, a substituted 3-aminocrotononitrile of Formula (IV) is reacted with an alcohol or mercaptan in an inert liquid medium containing an excess of hydrogen chloride gas (up to a 30 mole excess). The reaction is run at a temperature in the range of about -10°C to +50°C, preferably at about -10° to +30°C. In order to lessen or prevent side reactions, an excess of up to 4 moles of alcohol or mercaptan can be used, preferably about a 0.5 to 1 mole excess. Also, in order to lessen or prevent side reactions, the hydrogen chloride gas and the alcohol or mercaptan can be added first to the liquid medium and then the substituted 3-aminocrotononitrile (IV) can be added. Preferably, (IV) is added at a low temperture, about 0°C, and then the reaction mass is warmed to about room temperature. The liquid medium used in the reaction can be any inert liquid that provides suitable solubility for the reactants. Suitable liquids include chloroform, dioxane, methanol and methyl acetate. Methyl acetate is the preferred solvent. The reaction time is ordinarily about 1 to 24 hours, preferably about 3 to 6 hours.

Reaction A can be further exemplified by the following procedure: To a solution of about 400 ml methyl acetate saturated with hydrogen chloride gas and containing 1.7 mole of alcohol or mercaptan cooled to about 0°C is added a solution containing 1.2 mole of a 3-aminocrotononitrile (IV) dissolved in methyl acetate. After addition is complete, cooling is removed and the reaction mixture is stirred at room temperature for about 1 to 24 hours, cooled, and the product (I) is isolated by filtration.

The starting substituted 3-aminocrotononitriles (IV) described herein are known in the art and can be prepared by several methods, examples of which are described in the following references: D. J. Brown, et al., J. Chem. Soc. Perkins Trans., 372 (1974); H. U. Sieveking, et al., Angew. Chem., 81, 431 (1969); H. Adkins, et al., J. Am. Chem. Soc., 64, 151 (1942); N. Goasdoue, et al., J. Organometal Chem., 71 (3), 325 (1974); and Fr. 1,377,891 (1964), (Chem. Abst. 62:9021g).

Reaction B illustrates the process for preparing compounds of the invention (II). According to this process, a two-step sequence is used which can be more fully illustrated as follows:

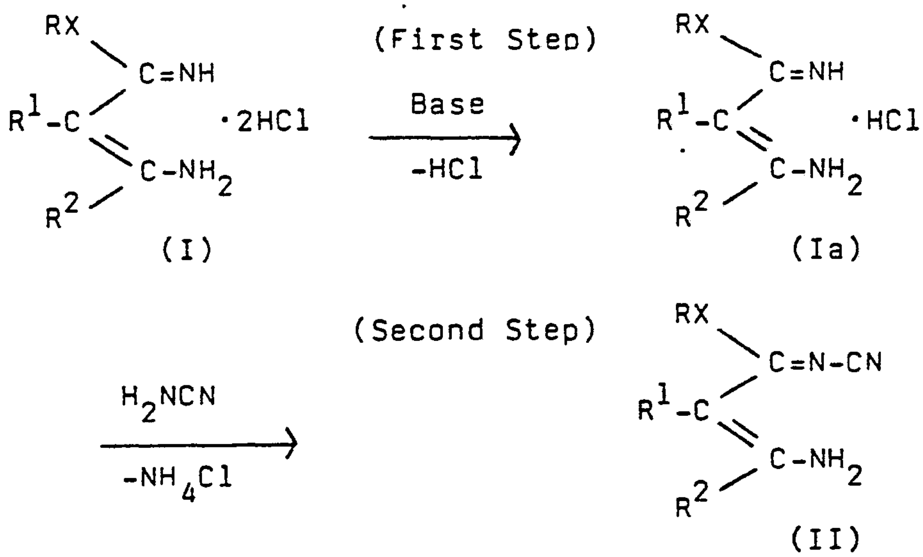


(I) (Ia) (II)

In the first step of Reaction B, the dihydrochloride salt of Formula (I) is contacted with one equivalent weight of a base in an inert liquid medium to produce a monohydrochloride salt (Ia). The pH should be no higher than about 7 when an aqueous medium is used. The base is preferably an alkali metal (preferably sodium) bicarbonate, carbonate, hydroxide or methoxide, pyridine or triethylamine (or other tertiary amine base). Sodium carbonate, sodium bicarbonate or sodium hydroxide are ordinarily used as their aqueous solutions. The liquid medium used to suspend or dissolve the reactants can be any liquid inert to the reaction. Typical liquids are halogenated compounds such as $CH_2Cl_2$ or $CHCl_3$, ethers such as diethyl ether or dioxane, esters such as methyl acetate or ethyl acetate, alcohols such as methanol or ethanol, hydrocarbons such as benzene, toluene, hexane and cyclohexane, and water. Methylene chloride and water are the preferred liquid mediums. The pH of an aqueous reaction mixture is preferably maintained in the range of 5-7. Temperature of the reaction is preferably in the range of about -10°C to about 20°C.

In the second step of Reaction B, the monohydrochloride salt (Ia) is contacted with cyanamide to produce a compound of the invention (II). Generally, a 0-80% molar excess of cyanamide is used, preferably as 50% aqueous cyanamide. The temperature of the reaction is usually in the range of about 10-40°C, preferably about 15-30°C. The reaction time is usually about 30 minutes to 2 hours. It is an important part of this invention that at higher temperatures or reaction times longer than about 2 hours, (II) can be further converted to pyrimidine (III), more fully described hereinafter.

Reaction B can be carried out by any one of the following procedures:

1) Step (1): solid dihydrochloride salt (I) is suspended in any one of the described organic liquid mediums and then contacted with one equivalent of base at -10° to 20°C to form monohydrochloride salt (Ia). Step (2): an excess (about 10 to 80% molar excess) of cyanamide, preferably as 50% aqueous cyanamide, is added and the reaction mass is warmed to 20° to 30°C where it is held for 30 minutes to 2 hours. Water is then added to dissolve the salts present. The aqueous phase is separated from the organic phase and (II) is isolated from the organic phase by evaporation of the solvent with vacuum at less than 30°C;

2) Step (1): solid dihydrochloride salt (I) is added to one equivalent of one of the preferred aqueous bases, e.g., $Na_2CO_3$, $NaHCO_3$ or NaOH, in water at -10° to 20°C to form monohydrochloride salt (Ia). Step (2): an excess (about 10 to 80% molar excess) of 50% aqueous cyanamide is added and the solution is warmed to room temperature where it is held for 30 minues to 2 hours. Solid product (II), which precipitates during this time, is then isolated by filtration;

3) Step (1): a suspension of dihydrochloride salt (I) in one of the described organic liquid mediums is added simultaneously with one equivalent of an aqueous base to ice-water, maintaining a pH of 5-7 by regulating the addition rate of either stream, to produce monohydrochloride salt (Ia). Step (2): an excess (about 10% to 80% molar excess) of 50% aqueous cyanamide is then added and the two-phase mixture warmed to room temperature where it is held for about 30 minutes to 2 hours. Water is then added to dissolve any salts present, and the reaction is worked up as described in 1) to produce product (II);

4) solid dihydrochloride salt (I) is suspended in methanol and then contacted with one equivalent of sodium methoxide at -10° to 10°C to form monohydrochloride salt (Ia). The salt (Ia) is isolated as a precipitate by (a) filtering the mixture to remove the NaCl formed, (b) evaporating the methanol from the filtrate under vacuum at less than 30°C, (c) adding a suitable organic liquid such as acetone, methylacetate or diethyl ether to precipitate (Ia), and (d) isolating (Ia) by filtration. Step (2): solid (Ia) is then suspended in one of the described organic liquid mediums and contacted with an excess (about 10 to 80% molar excess) of cyanamide, preferably as 50% aqueous cyanamide, and the reaction mass is stirred at room temperature for a period of 30 minutes to 2 hours. Water is then added and the mixture is worked up as described in 1) to produce product (II). The preferred procedure is 1) or 2) because of ease of synthesis and higher yields of product (II).

Reactions C, D and E illustrate the processes of this invention for preparing pyrimidines of Formula (III) which are useful as herbicide intermediates.

As illustrated in Reaction C, compounds of the invention (II) can be caused to undergo ring closure to pyrimidines (III) by heating. Preferably, compound (II) is dissolved or suspended in a suitable inert liquid medium such as water, methanol, toluene or xylene and maintained at a temperature in the range of about 0 to 140°C, preferably about 40-110°C, until the rearrangement is complete. Alternatively, ring closure can be accomplished by heating the neat compound of Formula (II) to a temperature above its melting point for a suitable period of time.

Reaction C is exemplified by one of the following procedures:

1) a solution or suspension of (II) in an inert liquid medium is heated to 40-110°C until rearrangement is complete (about 1 to 3 hours at 40°C to about 30 minutes at 110°C); then the solution or suspension is cooled and pyrimidine (III) isolated by filtration or evaporation of solvent;

2) a compound of Formula (II) is heated neat to its melting point, e.g., when R and $R^2$ are methyl and $R^1$ is H and X is O, heating to about 120°C will give ring closure in less than one minute. The ring closure reaction of either procedure 1) or 2) is highly exothermic and can lead to boiling of the liquid or a considerable rise in temperature of the melt when (II) is heated neat.

Alternatively, as illustrated in Reaction D, dihydrochloride salt (I) can be converted directly to pyrimidine (III). The process can be considered to occur as follows:

$$(I) \xrightarrow[H_2NCN]{\text{base}} \left[ (Ia) \rightarrow (II) \right] \rightarrow (III)$$

(Intermediates (Ia) and (II) are already described in Reaction B).

According to this process, dihydrochloride salt (I) is contacted with one equivalent weight of a base at a pH no higher than about 7 in an inert liquid medium, followed by the addition of cyanamide. The resultant reaction mass is then heated to a temperature in the range of about 25° to 100°C for a time sufficient to produce pyrimidine (III) (up to 24 hours at 25°C and to about one hour at 100°C). The base is ordinarily contacted with the dihydrochloride salt (I) at a temperature in the range of about -10° to 20°C. Typical bases that can be used include alkali metal (preferably sodium) bicarbonate, hydroxide or methoxide, pyridine or triethylamine (or other tertiary amine bases). Sodium carbonate, sodium bicarbonate or sodium hydroxide are usually used as their aqueous solutions. The preferred bases are pyridine and triethylamine (or other tertiary amine bases). Suitable inert liquid mediums in which the reaction can be run include halogenated compounds such as $CH_2Cl_2$ or $CHCl_3$, ethers such as dioxane or tetrahydrofuran, esters such as methyl acetate or ethyl acetate, hydrocarbons such as benzene or toluene, and water. The preferred liquid mediums are methylene chloride, tetrahydrofuran, methyl acetate and toluene. Preferably, an equivalent or slight excess (about 10%) of cyanamide is used in the reaction. The cyanamide can be added to the reaction mass as a solid, as a solution or suspension in one of the described organic liquid mediums, or as a 50% aqueous solution. The above-mentioned pH limitation, as elsewhere herein, is to be understood as applying to aqueous systems.

Reaction D is more fully exemplified by one of the following procedures:

1) solid dihydrochloride (I) is suspended in any one of the described non-aqueous liquid mediums and one equivalent of a base is added. After a few minutes, one equivalent or a slight excess (about 10%) of cyanamide is added, and the reaction mass is heated at about 40-80°C until the reaction is complete (up to 5 hours at 40°C and to 2 hours at 80°C). Water is then added to dissolve any salts present. Any undissolved product (III) is isolated by filtration, then the aqueous phase is separated from the organic phase and additional (III) is isolated from the organic phase by evaporation of the solvent at reduced pressure;

2) The procedure in 1) above is carried out except that one equivalent or a slight excess (about 10%) of 50% aqueous cyanamide is used;

3) solid dihydrochloride salt (I) is added to one equivalent of a base in water at about -10° to 10°C, followed by one equivalent or slight excess (about 10%) of 50% aqueous cyanamide. The resultant reaction mass is warmed to room temperature where it is held for about 2 hours and then heated at a temperature in the range of 25°-100°C until the reaction is complete (up to 24 hours at 25°C to about 1 hour at 100°C). After cooling, solid product (III) which precipitates during this time, is isolated by filtration. The preferred procedure is 1) or 2) above because of ease of synthesis and generally higher yields of product (III).

An alternate process for preparing pyrimidine (III) is illustrated in Reaction E, and is more fully described as follows:

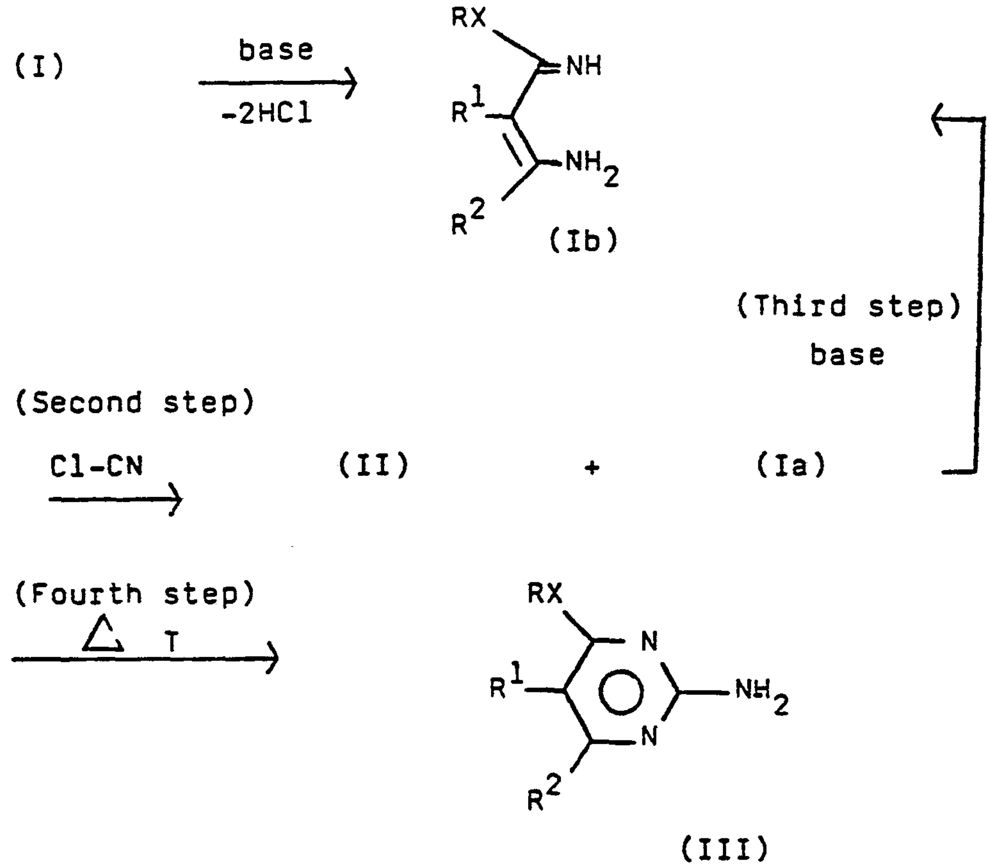


In the first step, dihydrochloride salt (I) is contacted with two equivalents of a base at a pH no higher than about 8 in an inert liquid medium to form imidate (Ib). The base is preferably an alkali metal or alkaline earth carbonate or hydroxide and more preferably an alkali metal carbonate such as sodium or potassium carbonate. Preferably, the salt (I), which is suspended in an inert organic liquid medium, is added to an aqueous solution of the base. Suitable liquid mediums include $CHCl_3$ and $CH_2Cl_2$, the latter being preferred. The aqueous base to solvent ratio is preferably about 1:1 although higher or lower ratios are operable. The reaction mixture is cooled to maintain a temperature within the range of about -10° to +50°C. The imidate product is contained in the organic phase.

The first step of Reaction E is exemplified by the following procedure: A suspension of (I) in one of the described liquid mediums is added to a solution or suspension of at least two equivalents of an aqueous base in ice-water. The organic phase containing the product (Ib) is removed and the water layer is further extracted with additional organic solvent. The combined organic solutions are dried over magnesium sulfate or sodium sulfate. If a different solvent is to be used in the first step, evaporation of solvent at reduced pressure will yield an oily product.

In the second step, imidate (Ib) is contacted with cyanogen chloride in an inert liquid medium to prepare a mixture of the monohydrochloride salt (Ia) and N-cyanoimidate (II). The reactants are combined in the organic layer retrieved from the first step, or, if the imidate has been isolated, in an inert organic solvent. Suitable solvents include methylene chloride, chloroform, tetrahydrofuran and dioxane, with methylene chloride being preferred. The cyanogen chloride is preferably in the amount of about one-half to about one mole per mole of imidate. The reaction is run at a temperature of about 0°C to 80°C, preferably about 20° to 45°C. When the solvent used is methylene chloride, pressure must be applied to achieve temperatures above reflux. The reaction time will vary with the temperature selected. For example, at the reflux temperature of methylene chloride, a reaction time of approximately 3 hours is required.

The monohydrochloride salt (Ia) precipitates during the reaction. Thus, the N-cyanoimidate (II) may be isolated by filtration followed by evaporation of the solvent.

As part of the process, it is recognized that for some thermally unstable N-cyanoimidates (II), ring closure to pyrimidines (III) may occur, either partially or totally, during the heating phase of the second step. For those cases, pyrimidine (III) and N-cyanoimidate (II) are isolated by (a) filtering and evaporating the filtrate to obtain N-cyanoimidate (II) plus any pyrimidine (III) dissolved in the solvent, and (b) isolating any insoluble pyrimidine (III) by washing the precipitate with water to remove the water soluble monohydrochloride (Ib).

It is also recognized, as part of the process, that isomers can form when cyanogen chloride is reacted with imidate (Ib), as illustrated by formulas (II) and (IIa).

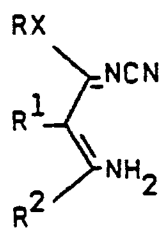

(II)

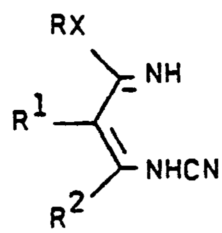

(IIa)

The presence of isomers is not critical, however, because both isomers can ring close to pyrimidine (III):

II, IIa $\longrightarrow$ III

The second step of Reaction E is exemplified by the following procedure:

Gas or liquid cyanogen chloride is added to the solution of (Ib) prepared in the first step. Alternatively, solvent-free (Ib) is dissolved or suspended in an inert liquid medium to which cyanogen chloride is added. The mixture is heated, and a precipitate of monohydrochloride salt (Ia) is formed. The mixture is cooled to 25°C and filtered. The filtrate, which contains the desired N-cyanoimidate (II), plus any solvent-soluble pyrimidine (III) formed during the reaction, is concentrated at reduced pressure to afford (II) plus (III). The precipitate isolated during the filtration procedure is washed with water to remove the monohydrochloride salt (Ia), leaving any insoluble pyrimidine (III) formed during the reaction.

The monohydrochloride salt (Ia) formed during the reaction may be recycled to produce the imidate (Ib) by following the procedure of the first step. Unlike the first step, however, only one equivalent of base is needed to neutralize the salt.

In the fourth step of Reaction E, the N-cyanoimidate (II) or mixture of (II) plus pyrimidine (III) is converted to pyrimidine (III) by heating. Preferably, the N-cyanoimidate or mixture of N-cyanoimidate plus pyrimidine is dissolved or suspended in a suitable inert liquid medium such as water, methanol, toluene or xylene and maintained at a temperature in the range of about 0° to 110°C, preferably about 40°C to 80°C, until the rearrangement is complete. Alternatively, ring closure can be accomplished by heating the neat compound or mixture of compounds (II) and (III) to a temperature above the melting point of (II) for a suitable period of time.

The fourth step of Reaction E is exemplified by the following procedure:

1) a solution or suspension of (II), or (II) plus (III), in an inert liquid medium is heated at 40-110°C until rearrangement is complete (several hours at 40°C to about 30 minutes at 110°C). Then the solution or suspension is cooled and the pyrimidine (III) is isolated by filtraton or evaporation of the solvent;

2) a compound of Formula (II), or a mixture of (II) plus a compound of Formula (III), is heated neat to the melting point of (II). For example, when X is O, R and $R^2$ are $CH_3$ and $R^1$ is H, heating to about 120°C will give ring closure in less than one minute.

Because of ease of synthesis and generally higher yields of product, the preferred process for preparing pyrimidine (III) is the process described as Reaction D.

By using the procedure described hereafter in Example 1, the compounds of Formula (I) listed in Table I can be prepared.

Table I

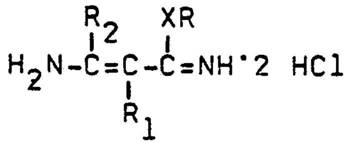

| R | $R_1$ | $R_2$ | X | m.p.(°C) |
|---|---|---|---|---|
| $CH_3$ | H | $CH_3$ | O | 99-102°C |
| $CH_2CH_3$ | H | $CH_3$ | O | |
| $CHCH_3$<br>$CH_3$ | H | $CH_3$ | O | |
| $CH_2CH_2CH_3$ | H | $CH_3$ | O | |
| $CH_2CH_2CH_2CH_3$ | H | $CH_3$ | O | |
| $CH_2CH_2OCH_3$ | H | $CH_3$ | O | |
| $CH_2OCH_2CH_3$ | H | $CH_3$ | O | |
| $CH_2OCHCH_2CH_3$<br>$CH_3$ | H | $CH_3$ | O | |
| $CH_2CH_2Cl$ | H | $CH_3$ | O | |
| $CH_2CF_3$ | H | $CH_3$ | O | |
| $CH_3$ | H | $CH_3$ | S | |
| $CH_2CH_3$ | H | $CH_3$ | S | |
| $CH_3$ | $CH_3$ | $CH_3$ | O | |
| $CH_3$ | $CH_2CH_3$ | $CH_2CH_2CH_3$ | O | |
| $CH_3$ | $CH_3$ | $CH_2CH_3$ | O | |
| $CH_3$ | H | $CH_2CH_2CH_3$ | O | |
| $CH_3$ | H | H | O | |
| $CHCH_2CH_3$<br>$CH_3$ | H | $CH_3$ | O | |
| $CH_2CHCH_3$<br>$CH_3$ | H | $CH_3$ | O | |
| $CH_3$ | $CH_2CH_2CH_3$ | $CH_3$ | O | |
| $C(CH_3)_3$ | H | $CH_3$ | O | |

Table I (continued)

| R | $R_1$ | $R_2$ | X | m.p.(°C) |
|---|---|---|---|---|
| $CH_2OCH_2CH_2CH_3$ | H | $CH_3$ | O | |
| $CH_2CH_2OCH_2CH_3$ | H | $CH_3$ | O | |
| $CH_2CH_2OCH_2CH_2CH_3$ | H | $CH_3$ | O | |
| $CH_2CH_2OCH_2CH_2CH_2CH_3$ | H | $CH_3$ | O | |
| $CH_2CH_2OCHCH_2CH_3$<br>\|<br>$CH_3$ | H | $CH_3$ | O | |
| $CH_2CH_2OCH_2CH(CH_3)_2$ | H | $CH_3$ | O | |

By using the procedures described hereafter in Examples 2 and 3, the compounds of Formula (II) listed in Table II can be prepared.

Table II

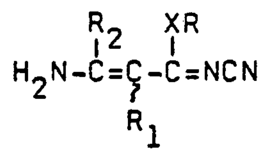

| R | $R_1$ | $R_2$ | X | m.p.(°C) |
|---|---|---|---|---|
| $CH_3$ | H | $CH_3$ | O | 123-124° |
| $CH_2CH_3$ | H | $CH_3$ | O | |
| $CHCH_3$<br>$CH_3$ | H | $CH_3$ | O | |
| $CH_2CH_2CH_3$ | H | $CH_3$ | O | |
| $CH_2CH_2CH_2CH_3$ | H | $CH_3$ | O | |
| $CH_2CH_2OCH_3$ | H | $CH_3$ | O | |
| $CH_2OCH_2CH_3$ | H | $CH_3$ | O | |
| $CH_2OCHCH_2CH_3$<br>$CH_3$ | H | $CH_3$ | O | |
| $CH_2CH_2Cl$ | H | $CH_3$ | O | |
| $CH_2CF_3$ | H | $CH_3$ | O | |
| $CH_3$ | H | $CH_3$ | S | |
| $CH_2CH_3$ | H | $CH_3$ | S | |
| $CH_3$ | $CH_3$ | $CH_3$ | O | |
| $CH_3$ | $CH_2CH_3$ | $CH_2CH_2CH_3$ | O | |
| $CH_3$ | $CH_3$ | $CH_2CH_3$ | O | |
| $CH_3$ | H | $CH_2CH_2CH_3$ | O | |
| $CH_3$ | H | H | O | |
| $CHCH_2CH_3$<br>$CH_3$ | H | $CH_3$ | O | |
| $CH_2CHCH_3$<br>$CH_3$ | H | $CH_3$ | O | |
| $CH_3$ | $CH_2CH_2CH_3$ | $CH_3$ | O | |
| $C(CH_3)_3$ | H | $CH_3$ | O | |

Table II (continued)

| R | $R_1$ | $R_2$ | X | m.p.(°C) |
|---|---|---|---|---|
| $CH_2OCH_2CH_2CH_3$ | H | $CH_3$ | O | |
| $CH_2CH_2OCH_2CH_3$ | H | $CH_3$ | O | |
| $CH_2CH_2OCH_2CH_2CH_3$ | H | $CH_3$ | O | |
| $CH_2CH_2OCH_2CH_2CH_2CH_3$ | H | $CH_3$ | O | |
| $CH_2CH_2OCHCH_2CH_3$<br>$\quad\quad\quad\quad\quad |$<br>$\quad\quad\quad\quad CH_3$ | H | $CH_3$ | O | |
| $CH_2CH_2OCH_2CH(CH_3)_2$ | H | $CH_3$ | O | |

As illustrated in Reaction F, it has also been discovered that herbicides of Formula (VI) are prepared in good yields when N-cyanoimidates (II) are contacted with arylsulfonylisocyanates (V). According to the process, the aryl group of the sulfonylisocyanate may be a benzaromatic, naphthylaromatic or heteroaromatic ring, such as 2-methoxycarbonylphenyl-, 2-chloronaphthalen-1-yl-, 2-chloropyridin-3-yl-, and 2-methoxycarbonylthien-3-yl-.

The process comprises reacting the N-cyanoimidate (II) with one equivalent or slight excess (5% to 10%) of arylsulfonylisocyanate (V) in an inert liquid medium at 10°C to 50°C for 5 minutes to 3 hours. The pyrimidino arylsulfonylureas (VI) formed are readily isolated by filtration or evaporation procedures. Suitable inert liquid mediums include halocarbons such as $CH_2Cl_2$ or $CHCl_3$, ethers such as dioxane or tetrahydrofuran, hydrocarbons such as toluene and xylene, esters such as ethyl acetate or methyl acetate and acetonitrile.

Reaction F is exemplified by the following procedure:

1) to a solution or suspension containing N-cyanoimidate (II) is added at 0°C to 30°C a solution containing one equivalent or a slight excess (about 10%) of arylsulfonylisocyanate (V); the reaction mixture is stirred at room temperature for 1 to 3 hours, and the sulfonylurea (VI) is isolated by filtration or evaporation of the solvent under reduced pressure.

As part of the invention, it is recognized that N-cyanoimidate (II) may be converted to pyrimidine (III) under the conditions of reactions described above. Any pyrimidine, preformed or formed under these conditions, will also react with the arylsulfonylisocyanate to form sulfonylurea (VI) in good yields, as illustrated heretofore in Reaction G.

The processes of this invention are further illustrated by the following examples in which parts and percentages are by weight unless otherwise indicated.

Example 1

A solution of 67.2 g methanol in 488 ml methyl acetate was cooled at 0°C and saturated with hydrogen chloride gas. Next, a solution of 100 g 3-aminocrotononitrile (85% pure) dissolved in 366 ml methyl acetate was slowly added while still cooling the reaction solution at 0°C. Cooling was then removed and the solution was stirred at room temperature 6 hours, with precipitation starting after about 0.5 hours of stirring. The mixture was then cooled to 5°C, filtered and the solid methyl 3-amino-2-butenimidate·2HCl was washed with excess methyl acetate and dried under nitrogen atmosphere; yield 178 g, m.p. 99-102°C.

Analysis-Calculated for $C_5H_{12}N_2Cl_2O$: C, 32.1; H 6.5; N, 14.9.

Found: C, 31.7; H, 6.1; N, 14.9.

Example 2

To a suspension of 12.6 g $NaHCO_3$ in 130 ml water cooled at 0°C was added portionwise 30 g of methyl 3-amino-2-butenimidate·2HCl (Example 1). To the resulting solution was added in one portion 25 g of 50% aqueous cyanamide and the solution was warmed to room temperature where it was held for 45 minutes. Solid methyl 3-amino-N-cyano-2-butenimidate precipitated during this time which was removed by filtration, after having cooled the mixture to 5°C; yield 9 g, m.p. 114-117°C. A portion of the solid was recrystallized from acetone at less than 30°C by dissolving the solid in excess acetone and evaporating the solvent at room temperature under vacuum until precipitation started. The mixture was then cooled to 5°C and filtered to give pure methyl 3-amino-N-cyano-2-butenimidate; m.p. 123-125°C.

Analysis-Calculated for $C_6H_9N_3O$: C, 51.8; H, 6.5; N, 30.2.

Found: C, 51.9; H, 6.5; N, 30.1.

Example 3

To a suspension of 9.4 g methyl 3-amino-2-butenimidate dihydrochloride in 100 ml methylene chloride was added dropwise 5.1 g triethylamine while cooling the suspension at 5-15°C. After stirring the mixture for several minutes, 6.5 g of 50% aqueous cyanamide was added in one portion. The mixture was then warmed to room temperature where it was stirred for 1.5 hours, then diluted with 25 ml of water. The organic phase was separated, dried over $Na_2SO_4$, and evaporated under vacuum at less than 30°C to give crude methyl 3-amino-N-cyano-2-butenimidate; yield 3.8 g, m.p. 115-118°C.

Example 4

One gram of crude product from Example 2 was heated in a test tube with the aid of an oil bath to about 120°C, at which point a sudden release of energy raised the temperature of the melt to 175°C. The melt was then cooled and solidified. The resulting product melted at 155-158° and its IR spectrum was identical with pure 2-amino-4-methoxy-6-methylpyrimidine.

Example 5

A suspension of 4 g of crude product from Example 2 in methanol was heated at 50-60°C for 30 minutes. The methanol was evaporated from the mixture to give 2-amino-4-methoxy-6-methylpyrimidine in quantitative yield, m.p. 155-158°C.

Example 6

To a suspension of 9.4 g methyl 3-amino-2-butenimidate dihydrochloride in 65 ml of methylene chloride was added dropwise 5.1 g of triethylamine while cooling the suspension at about 5-20°C. After stirring the resultant mixture about 5 minutes, a suspension of 2.1 g cyanamide in 10 ml of methylene chloride was added and the mixture was heated at 40°C for 2 hours, then cooled to room temperature and diluted with 30 ml of water. Solid product, 2-amino-4-methoxy-6-methylpyrimidine, which precipitated during the reaction, was removed by filtration. Additional product was obtained by separating the aqueous phase from the filtrate and evaporating the organic phase to dryness. Solid product from the filtration and evaporation procedures were combined and dried to yield 6 g of 2-amino-4-methoxy-6-methylpyrimidine; m.p. 154-157°C.

Example 7

The procedure in Example 6 was repeated using 4.5 g of 50% aqueous cyanamide instead of a suspension of cyanamide in methylene chloride. The mixture was heated at 40°C for 2 hours. This reaction yielded 5.4 g of solid product, 2-amino-4-methoxy-6-methylpyrimidine; m.p. 154-157°C.

Example 8

To a suspension of 12.6 g sodium bicarbonate in 130 ml water at 0°C was added, in small portions, at a pH greater than 4.5, 30 g of methyl 3-amino-2-butenimidate dihydrochloride (Example 1). To the resulting solution was added 13.5 g of 50% aqueous cyanamide and the solution was warmed to room temperature where it was held for 16 hours. Solid product, 2-amino-4-methoxy-6-methylpyrimidine, which precipitated during this time, was removed by filtration and dried; yield 12 g, m.p. 155-158°C. The product was contaminated with a small amount of high melting impurity which was removed by washing the product with warm 1N aqueous NaOH.

Example 9

The reaction in Example 8 was repeated except that the reaction mixture was stirred at room temperature for 2 hours and then heated at 100°C for one hour. The reaction mixture was then cooled and filtered to give 11 g of 2-amino-4-methoxy-6-methylpyrimidine; m.p. 155-158°C. A small amount of high melting impurity was removed by washing the product with warm 1N aqueous NaOH.

Example 10

To a stirred mixture of 400 ml aqueous potassium carbonate (300 g/liter) and 400 ml methylene chloride, 73 g of methyl 3-amino-2-butenimidate dihydrochloride is added in portions. The organic layer is removed, and the aqueous layer is extracted with three-80 ml portions of methylene chloride. The combined organic layers are dried over anhydrous sodium sulfate and then filtered. The filtrate is evaporated to give methyl 3-amino-2-butenimidate as an oil.

Example 11

A 3.5 ml portion of cyanogen chloride is added to a solution of 7.4 g of the product of Example 10 in 100 ml of methylene chloride. After the mixture is refluxed for 4 hours, the solvent is evaporated. The residue is washed with water to give crude 2-amino-4-methoxy-6-methylpyrimidine. The yield is increased by recycling the methyl 3-amino-2-butenimidate monohydrochloride by-product dissolved in the water washing.

Example 12

To a suspension of 2 g of methyl 3-amino-N-cyano-2-butenimidate in 25 ml acetonitrile was added slowly a solution of 3 g of 2-chlorobenzenesulfonylisocyanate in 5 ml of acetonitrile. A mild exotherm occurred (25°-35°C) as a solution formed followed by a precipitate within 5 minutes after the addition. The mixture was stirred at room temperature for 2 hours, cooled to 5°C and filtered to give the solid product, 2-chloro-N-[(4-methoxy-6-methylpyrimidin-2-yl)aminocarbonyl]benzenesulfonamide; yield 4 g, m.p. 204-206°C.

Claims:

1. A compound of the formula:

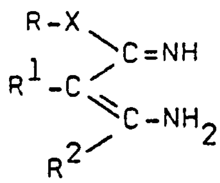

and the monohydrochloride and dihydrochloride thereof, wherein

X is O or S;

R is $C_1$-$C_4$ alkyl, $(CH_2)_n$-$OR^3$, $CH_2CH_2Cl$ or $CH_2CF_3$;

$R^1$ is H or $C_1$-$C_3$ alkyl;

$R^2$ is H or $C_1$-$C_3$ alkyl;

$R^3$ is $C_1$-$C_4$ alkyl; and

n is 1 or 2

provided that when R is $CH_2CH_2Cl$ or $CH_2CF_3$, then X is O.

2. A compound of the formula

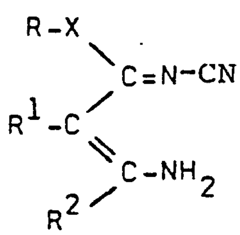

or

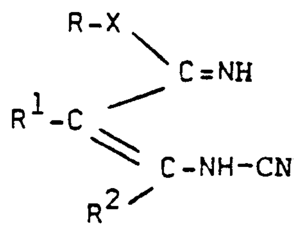

wherein X, R, $R^1$, $R^2$, $R^3$ and n are as defined in claim 1.

3. A compound of claim 1 or 2 in which X is O, $R^1$ is H and $R^2$ is $CH_3$.

4. A compound of claim 3 in which R is $C_1$-$C_4$ alkyl.

5. A compound of claim 1 which is methyl 3-amino-2-butenimidate and its monohydrochloride and dihydrochloride.

6. A compound of claim 2 which is methyl 3-amino-N-cyano-2-butenimidate.

7. A process for preparing a compound of claim 1

which comprises contacting a nitrile of the formula

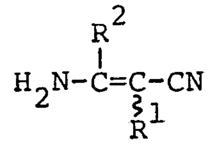

wherein $R^1$ and $R^2$ are as defined in claim 1, with an alcohol or mercaptan of the formula

RXH

wherein R and X are as defined in claim 1, in an inert liquid medium containing an excess of up to 30 moles of hydrogen chloride gas and an excess of up to 4 moles of said alcohol or mercaptan, at a temperature of $-10^o$ to $+50^oC$.

8. The process of claim 7 wherein the inert liquid medium is methyl acetate.

9. The process of claim 8 wherein a methyl acetate solution containing the nitrile is contacted with a methyl acetate solution saturated with hydrogen chloride gas and containing a 0.5 to 1 mole excess of RXH, at a temperature in the range of $-10^oC$ to $10^oC$, and the resultant reaction mass is stirred at a temperature in the range of $10^oC$ to $30^oC$.

10. A process for preparing a compound of claim 2 which comprises contacting the monohydrochloride salt of a compound of claim 1 with cyanamide or contacting the free base of a compound of claim 1 with cyanogen chloride.

11. The process of claim 10 wherein cyanogen chloride is employed and wherein the imidate monohydrochloride by-product is contacted with base to form said compound of claim 1, which is then reacted with further cyanogen chloride.

12. A process for preparing a compound of claim 2 comprising:

a) contacting a dihydrochloride salt of the formula:

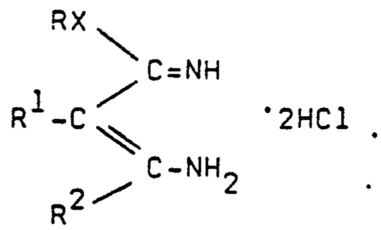

wherein X, R, $R^1$ and $R^2$ are as defined in claim 2, with an inert liquid medium containing about one equivalent weight of a base, the pH being no higher than about 7 when said medium is aqueous, to produce a monohydrochloride salt; and

b) contacting the monohydrochloride salt with cyanamide.

13. The process of claim 12 wherein the inert liquid medium is methylene chloride or water and said base is an alkali metal carbonate, bicarbonate, hydroxide or methoxide; pyridine or triethylamine.

14. The process of any of claims 10 to 13 wherein the resulting compound is heated at a temperature sufficient to cause ring closure and form a compound of the formula:

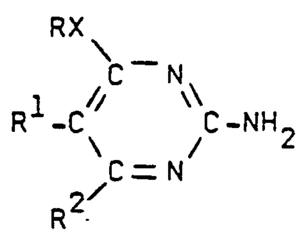

15. A process for preparing a compound of the formula:

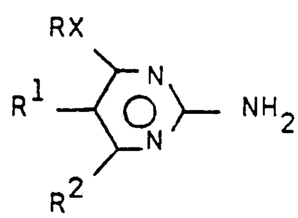

wherein R, $R^1$, $R^2$ and X are as defined in claim 1, which comprises heating a compound of claim 2 at a temperature sufficient to cause ring closure.

16. The process of claim 15 wherein the compound is heated (a) neat at a temperature above its melting point or (b) in an inert liquid medium selected from water, methanol, xylene or toluene at a temperature in the range of 45-110°C, for a time sufficient to complete ring closure.

17. A process for preparing the compound of the formula:

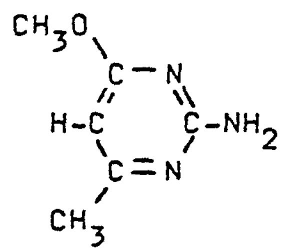

comprising:

a) contacting a suspension of the dihydrochloride salt of the formula:

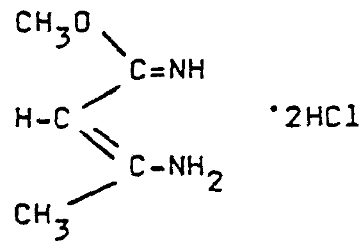

,

in methylene chloride with two mole equivalents of sodium or potassium carbonate at a temperature in the range of -10°C to 25°C to produce a free imidate; and

b) contacting a solution of the imidate in methylene chloride with cyanogen chloride at a temperature of 0° to 40°C to produce a mixture of a monohydrochloride imidate salt and cyanoimidates of the formulas:

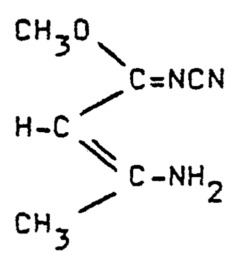

and

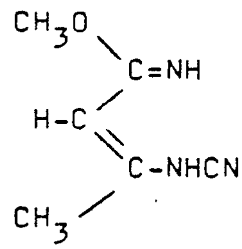

c) isolating the cyanoimidates from the reaction mixture;

d) heating the cyanoimidates in methylene chloride or toluene at a temperature sufficient to ring closure; and

e) recycling the monohydrochloride imidate salt by:

i) contacting the monohydrochloride imidate salt with one mole equivalent of sodium or potassium carbonate under the conditions of step (a) to produce a free imidate, and

ii) contacting the imidate with cyanogen chloride under the conditions of step (b).

18. A process for preparing a compound of the formula:

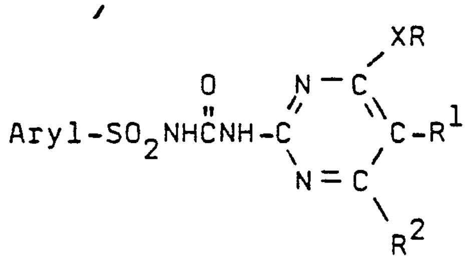

wherein R, $R^1$, $R^2$ and X are as defined in claim 1 and Aryl represents a benzaromatic, naphthylaromatic or heteroaromatic ring; which comprises contacting a compound of claim 2 in an inert liquid medium with a compound of the formula:

$$Ar\text{-}SO_2NCO$$

wherein Aryl is as defined above.

19. The process of claim 18 wherein the inert liquid medium is methylene chloride, xylene, toluene, acetonitrile,

methyl or ethyl acetate or tetrahydrofuran and the temperature is in the range of 20-50°C.